# EUROPEAN PATENT APPLICATION

(11) **EP 4 778 484 A1**
(43) Date of publication of application: **22.07.2026**
(21) Application number: 25165289.7
(22) Date of filing: 21.03.2025
(51) Int. Cl.: A61B 18/20, A61N 5/06, A61N 5/067, A61B 18/00

(54) **DERMATOLOGICAL LASER APPARATUS FOR DERMATOLOGICAL TREATMENTS**

(30) Priority: 16.01.2025 US 202563745847 P
(71) Applicant: Advalight ApS, 2750 Ballerup (DK)
(72) Inventor: KOTHARE, Amogh, 2750 Ballerup (DK); SCOPELLITI, Matteo Giuseppe, 2750 Ballerup (DK); MORTENSEN, Jesper Liltorp, 2750 Ballerup (DK); THORHAUGE, Morten, 2750 Ballerup (DK)
(74) Representative: Guardian IP Consulting I/S

(57) **Abstract**

A dermatological laser apparatus for dermatological treatment of a patient's skin using a treatment laser beam, the dermatological laser apparatus comprising a laser module, a beam delivery device, and a control circuit, wherein the laser module is configured to create bursts of laser radiation, each burst comprising laser radiation at least one of a set of laser wavelengths, the set comprising two or more laser wavelengths, wherein the beam delivery device is configured to direct the created laser radiation as a treatment laser beam towards a target area of the patient's skin; and wherein the control circuit is configured to: obtain information about the patient's skin type; select, in dependence of at least the patient's skin type, relative amounts of laser energy to be delivered at respective ones of the set of laser wavelengths; and control the laser module to create the bursts of laser radiation such that each burst comprises the selected relative amounts of laser energy at the respective ones of the set of laser wavelengths.

## Description

### Technical Field

The present disclosure relates to a dermatological laser apparatus for dermatological treatment of a patient's skin using a treatment laser beam, and to a method for controlling a dermatological laser apparatus.

### Background

Skin, in particular human skin, can be affected by a range of biological and ageing effects and by environmentally induced damages, such as wrinkles, acne, sun damage, reddening, vascular disorders and scarring. Rejuvenation is the combined area of treating a variety of conditions in order to restore the youthful appearance of the skin. One of the preferred treatments for skin rejuvenation is light-based treatment, also referred to as photo rejuvenation. Also other skin conditions can be treated using light therapy.

Various dermatological laser systems for treatment of skin conditions are known in the art. Various systems use a handheld device for directing a laser beam onto the skin area to be treated.

For example, US 2011/0306955 discloses a laser system for skin treatment. The laser system comprises a first laser resonator comprising at least a first gain medium for generating a first optical field along a first optical axis. The first laser resonator further comprises a first reflective element and a partly reflective first output coupler. The laser system also comprises a second laser resonator comprising at least a second gain medium for generating a second optical field along a second optical axis. The second laser resonator further comprises a second reflective element and a partly reflective second output coupler. The laser system comprises at least one nonlinear medium for generating a third optical field along a third optical axis by a nonlinear interaction between the first optical field and the second optical field. Finally, the laser system comprises an optical output port capable of delivering the first optical field, the second optical field, and the third optical field to an output, and at least one optical pump source for optically pumping the first gain medium and the second gain medium. The above prior art disclosure recognizes that different skin conditions can advantageously be treated by different wavelengths. As different wavelengths have different penetration depths in the skin and target different chromophores, treatments may beneficially utilize two or more of the wavelengths available from the laser system.

However, it remains desirable to provide a dermatological laser apparatus that facilitates safe and efficient treatment of a wide variety of different patients. In particular, it is desirable to provide a dermatological laser apparatus that facilitates efficient treatment of skin conditions while afflicting little discomfort to the patient being treated.

It is further desirable to provide a dermatological laser apparatus that is easy to operate.

It is further desirable to provide a dermatological laser apparatus that is safe.

It is further desirable to provide a dermatological laser apparatus that is reliable.

It is further desirable to provide a dermatological laser apparatus that is capable of performing a variety of types of treatments.

It is further desirable to provide a dermatological laser apparatus that can be manufactured and maintained at a relatively low cost.

In view of the above, it thus remains desirable to provide a dermatological laser apparatus and a control method for such a dermatological laser apparatus that address one or more of the above needs and/or other needs that exist in the field of dermatological laser apparatus, or to provide a dermatological laser apparatus and a control method that at least may serve as an alternative to existing solutions.

### Summary

According to one aspect, disclosed herein are embodiments of a dermatological laser apparatus for dermatological treatment of a patient's skin using a treatment laser beam, the dermatological laser apparatus comprising a laser module, a beam delivery device, and a control circuit, wherein the laser module is configured to create bursts of laser radiation, each burst comprising laser radiation at least one of a set of laser wavelengths, the set comprising two or more laser wavelengths, wherein the beam delivery device is configured to direct the created laser radiation as a treatment laser beam towards a target area of the patient's skin; and wherein the control circuit is configured to:
- obtain information about the patient's skin type,
- select, in dependence of at least the patient's skin type, relative amounts of laser energy to be delivered at respective ones of the set of laser wavelengths, and
- control the laser module to create the bursts of laser radiation such that each burst comprises the selected relative amounts of laser energy at the respective ones of the set of laser wavelengths.

Accordingly, the apparatus delivers laser energy at multiple wavelengths simultaneously, tailored to the patient's skin type and, optionally, tailored to the specific treatment indications, thereby ensuring maximum effectiveness and safety.

Laser energy at the multiple wavelengths is delivered during each burst, thereby avoiding the need for manual switching between wavelengths when performing treatments of skin conditions that benefit from exposure to different wavelengths.

The customizable wavelength selection and relative energy contribution per burst, based on the patient's skin type and, optionally, based on the treatment indication, enhances both efficacy and safety.

The present disclosure relates to different aspects, including the apparatus described above and in the following, further methods, systems, devices and product means, each yielding one or more of the benefits and advantages described in connection with one or more of the other aspects, and each having one or more embodiments corresponding to the embodiments described in connection with one or more of the other aspects described herein and/or as disclosed in the appended claims.

In particular, another aspect disclosed herein relates to embodiments of a method for controlling a dermatological laser apparatus for dermatological treatment of a patient's skin using a treatment laser beam, the method comprising:
- obtaining information about the patient's skin type,
- selecting, in dependence of the patient's skin type, relative amounts of laser energy to be delivered at respective ones of a set of laser wavelengths, and
controlling the dermatological laser apparatus to create bursts of laser radiation such that each burst comprises the selected relative amounts of laser energy at the respective ones of the set of laser wavelengths.

### Brief description of the drawings

The above and other aspects will be apparent and elucidated from the embodiments described in the following with reference to the drawing in which:
FIG. 1 schematically illustrates a block diagram of an embodiment of a dermatological laser apparatus.
FIG. 2 schematically illustrates a flow diagram of an embodiment of a process for controlling a dermatological laser apparatus.
FIG. 3 schematically illustrates an example of a laser module of an embodiment of a dermatological laser apparatus.
FIG. 4 illustrates the emission of bursts of pulsed laser radiation.

### Detailed description

In the following, embodiments of a dermatological laser apparatus and of a method of controlling such an apparatus will be described in more detail.

According to one aspect, disclosed herein are embodiments of a dermatological laser apparatus for dermatological treatment of a patient's skin using a treatment laser beam, the dermatological laser apparatus comprising a laser module, a beam delivery device, and a control circuit, wherein the laser module is configured to create bursts of laser radiation, each burst comprising laser radiation at least one of a set of laser wavelengths, the set comprising two or more laser wavelengths, wherein the beam delivery device is configured to direct the created laser radiation as a treatment laser beam towards a target area of the patient's skin; and wherein the control circuit is configured to:
- obtain information about the patient's skin type,
- select, in dependence of at least the patient's skin type, relative amounts of laser energy to be delivered at respective ones of the set of laser wavelengths, and
- control the laser module to create the bursts of laser radiation such that each burst comprises the selected relative amounts of laser energy at the respective ones of the set of laser wavelengths.

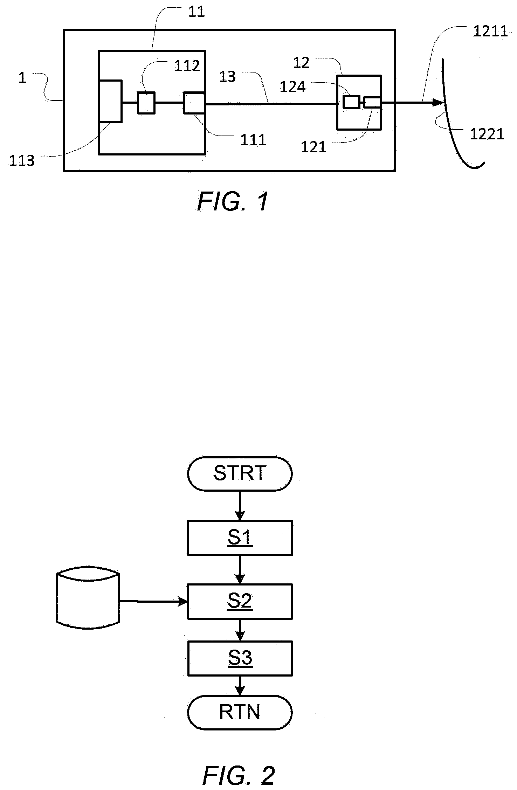

Skin type variation in patients results from their ability to produce and store varying amounts of melanin, the chromophore targeted when treating pigmentary conditions. In addition to being a direct target of laser- and light-based therapy, melanin is also the first chromophore to interact with light (during non-ablative, non-invasive treatments) owing to the superficial nature of its genesis and storage. Melanin absorbs strongly in the UV and visible regions of the light spectrum, and its absorption decays logarithmically in the NIR and IR regions, see e.g. Figure 2.4 in Sørensen Dam, J. (2000). Optical Analysis of biological media - continuous wave diffuse spectroscopy. [Doctoral Thesis (compilation), Atomic Physics]. Department of Physics, Lund University. As a result, for laser- and light-based devices deploying wavelengths in the visible region, the amount of energy delivered to create a similar thermal effect in the epidermal tissue varies depending on the amount of melanin present in the epidermis and, therefore, on the patient's skin type.

The temperature rise at the epidermal-dermal junction (EDJ) directly correlates with the safety of laser therapy. It is prudent for the temperature of the EDJ not to exceed 50 °C, as this thermal effect can cause dermal-epidermal separation, resulting in a blister (see e.g. Scopelliti, M. G., Kothare, A., & Karavitis, M. (2022). A novel 1726-nm laser system for safe and effective treatment of acne vulgaris. Lasers in Medical Science, 37(9), 3639-3647), and subsequently causing hyperpigmentation in darker skin types and hypopigmentation in lighter skin types.

Variation in energy delivery depending on the patient's skin type, via changes to the dosimetry at a particular wavelength or by deploying a wavelength that is not as aggressively absorbed by melanin, may result in a safer and more comfortable treatment without impacting efficacy.

Embodiments of the dermatological laser apparatus disclosed herein adjust the dosimetry based on the patient's skin type and, optionally, based on the particular indication to be treated. In particular, increased energy at longer wavelengths - at which melanin absorption is reduced - may be used for darker skin types.

The information about the patient's skin type may include any suitable measure of the amount of melanin pigment in the patient's skin and/or a measure of the skin's reaction to exposure to sunlight or otherwise. The information about the patient's skin type may include a classification of the skin type in one of a number of predetermined skin type classes, e.g. according to the Fitzpatrick skin type classification, also referred to as the phototype. The Fitzpatrick skin type (or phototype) is a numerical classification schema for human skin color, which depends on the amount of melanin pigment in the skin. This is determined by constitutional color (white, brown, or black skin) and the effect of exposure to ultraviolet radiation (tanning). The Fitzpatrick skin phototype is a constitutional characteristic present at birth. According to the Fitzpatrick classification, the skin type is classified as being one of six skin types, normally referred to as skin types I - VI, ranging from pale white skin to dark brown or black skin. Alternatively, the information about the skin type may include a classification according to another suitable classification scheme, e.g. the von Luschan chromatic scale or the Monk skin tone scale.

In some embodiments, the dermatological laser apparatus may comprise a user interface configured to provide functionality allowing a user to select a skin type and/or otherwise enter information about the skin type. The user interface may further provide functionality that allows the user to select an intended type of treatment or type of skin condition to be treated and/or one or more parameters associated with an intended type of treatment. The control unit may thus be configured to control the laser module based on the received user input. The user interface may include any suitable user interface device, e.g. a touch screen and/or a display and/or one or more user-input elements, such as one or more buttons, dials, knobs, joysticks, voice input devices, haptic input devices, etc.

The control unit selects, in dependence of at least the patient's skin type, relative amounts of laser energy to be delivered at respective ones of the set of laser wavelengths. To this end, the control unit may have stored thereon or have otherwise access to, stored treatment configuration data associating treatment types and skin types with laser module settings, where the laser module settings include information about relative laser energies at respective wavelengths. The stored treatment configuration data may include a look-up table associating skin types with respective relative amounts of laser energy at different wavelengths. Preferably, the control unit selects, in dependence of at least the patient's skin type and the type of skin condition to be treated, relative amounts of laser energy to be delivered at respective ones of the set of laser wavelengths. To this end, the look-up table may associate skin types and skin conditions with respective relative amounts of laser energy at different wavelengths.

An example of such a look-up table is shown in Table 1 below, where wavelength and dosimetry are adjusted based on the patient's skin type and indications. In the example of Table 1, the dermatological laser apparatus is operable to create bursts of laser radiation, each burst comprising laser radiation at at least one of a set of laser wavelengths, the set comprising three laser wavelengths, namely, in this example the wavelengths 589 nm, 1064 nm and 1319 nm.

**Table 1: Example of energy delivery tailored on patient's skin type and indication.**

| **Indication** | **Skin Type [I- VI]** | **Wavelengths Used** | **% Total Energy** |
|---|---|---|---|
| **Rosacea** | I - III | 589 | 100% |
| | IV-V | 589 | 60% |
| | | 1064 | 40% |
| | VI | 1064 | 100% |
| **Facial Telangectasia** | I - III | 589 | 100% |
| | IV-V | 589 | 65% |
| | | 1064 | 35% |
| | VI | 1064 | 100% |
| **Lentigo** | I - III | 589 | 100% |
| | IV-V | 589 | 50% |
| | | 1064 | 50% |
| | VI | 1064 | 100% |
| **Hyperpigmentation** | I - III | 589 | 60% |
| | | 1319 | 40% |
| | IV-V | 589 | 30% |
| | | 1064 | 30% |
| | | 1319 | 40% |
| | VI | 1064 | 60% |
| | | 1319 | 40% |
| **Acne** | I - III | 589 | 50% |
| | | 1319 | |
| | IV-V | 589 | 30% |
| | | 1064 | 20% |
| | | 1319 | 50% |
| | VI | 1064 | 50% |
| | | 1319 | 50% |

It will be appreciated that other examples of look-up tables may include alternative or additional skin conditions. Similarly, other examples may include a different set of wavelengths, e.g. sets with only two wavelengths or more than three wavelengths.

For example, in one embodiment, the lookup table includes the indications Acne Vulgaris, Benign Pigmented Lesions and Vascular lesions, and the set of wavelengths includes two wavelengths, e.g. 589 nm and 1319 nm.

It will further be appreciated that other embodiments may use different individual wavelengths.

It will further be appreciated that, in other embodiments, the control unit may include a different representation of the relationship between skin type and relative amounts of laser energy at different wavelengths.

The set of wavelengths may include a discrete number of wavelengths, e.g. representing center wavelengths of narrow frequency bands of laser radiation the laser module is configured to emit. The laser module may thus be configured to emit a discrete set of narrow-band wavelength components, each wavelength component having a center frequency and a bandwidth. The center wavelengths of the set of wavelength components may be spaced apart further than the widths of the frequency bands.

The user interface may include a display device configured to display proposed relative amounts of laser energy. Accordingly, the control unit may retrieve the proposed relative amounts of laser energy from the stored treatment configuration data, e.g. based on information about the patient's skin type entered by the user. The user interface may provide functionality that allows the user to accept and/or change the proposed relative amounts of energy.

In some embodiments, the dermatological laser apparatus is configured to irradiate the target skin area by one or more bursts of pulsed laser radiation. Each burst may comprise a pulse train of laser pulses, each laser pulse having one or more wavelength components suitable for heating constituents in the skin.

Examples of treatments that may be performed, and of skin conditions that may be treated, by some embodiments of the dermatological laser apparatus include, but are not limited to, one or more of the following skin conditions and treatments: inflammatory acne vulgaris, port wine stains, telangiectasia, such as facial, telangiectasia, rosacea, lentigo hemangioma, venous lake, angiomas, scars, striae, warts, fine lines and wrinkles, psoriasis, atrophic acne scars, spider veins, melasma, hyperpigmentation, skin rejuvenation. Accordingly, the apparatus is capable of performing a variety of treatments, each in an optimized manner while reducing discomfort to the patient.

Generally, in some embodiments, the dermatological treatments may be therapeutic treatments while, in other embodiments, the dermatological treatments may be non-therapeutic treatments, in particular cosmetic treatments.

It will be appreciated that the treatment configuration data may include additional treatment parameters and/or the user interface may provide functionality for allowing the user to enter or adapt other treatment parameters. Generally, examples of treatment parameters include: a wavelength of the treatment laser beam, an intensity of the treatment laser beam, a duration of exposure to the treatment laser beam, a shape and/or size of a target area across which the treatment laser beam is to be scanned, a scan pattern for scanning the treatment laser beam across a target area, and/or the like.

The radiation-delivery device may be a hand-held or hands-free radiation-delivery device, for directing the laser radiation as a treatment laser beam onto a target area of a surface of skin to be treated, thereby allowing efficient and flexible operation of the apparatus. The radiation-delivery device may comprise a body portion defining a beam output port for emitting the treatment beam along a beam aiming direction towards a target area. The beam output port may be defined by a suitable aperture, a lens and/or the like. For hand-held radiation delivery, the hand-held device may further comprise a guide member for assisting the user of the apparatus to position the hand-held radiation-delivery device relative to the skin surface to be treated. To this end, the guide member may extend from the hand-held body portion to a target distance from the beam output port along the beam aiming direction, e.g. such that a distal end of the guide member defines an appropriate distance of the skin to be treated from the output port. The distal end may thus be at a predetermined distance from the beam output port. The distal end may comprise a frame portion defining an aiming area on the skin to be treated when the hand-held radiation-delivery device is held with the frame portion touching the skin surface. For example, the frame portion may be an annular member or otherwise an element that surrounds an aiming area. The radiation-delivery device may thus be configured to direct the treatment beam towards one or more treatment spots within the aiming area, i.e. the target area of skin being treated by the treatment beam lies within the aiming area, preferably centered relative to the aiming area.

The laser module may be arranged in a housing separate from the radiation-delivery device. To this end, the radiation-delivery device may be optically connected to the laser module, preferably via a suitable fiber-optic cable, or otherwise. In this way, the optical output from the laser module may conveniently be delivered to a point of treatment on the skin of a patient. In some embodiments, the radiation-delivery device comprises at least one beam delivery mirror, in particular a scanning mirror. In this way, a particularly user-friendly dermatological laser apparatus may be achieved.

In some embodiments, the radiation-delivery device is not necessarily hand-held. Instead, the radiation-delivery device may e.g. have the form of a fixture or mounting element for attaching, mounting or fixating the radiation-delivery device relative to the target area, e.g. as a fixture to be attached or otherwise fixedly positioned relative to a patient's body.

In some embodiments, the laser module includes a laser resonator and a Q-switch, which may be active or passive. The laser resonator may include a suitable gain medium. The gain medium of the laser resonator may be a crystal based, and/or glass based, and/or fiber based medium. For example, crystal-based gain materials may be in the shape of slabs, rods, blocks, fibers or thin disks. Generally, the gain medium of the laser resonator may be configured to provide a gain at the desired wavelength. Furthermore, the gain medium may be configured to sustain the optical power level within a resonator of the laser source under operation.

In some embodiments, the gain medium comprises a rare-earth doped crystal. In alternative embodiments, the gain medium may be any of the following: a crystal, a gas gain medium, a dye gain medium, a crystal gain medium, a solid phase gain medium, or a semiconductor laser gain medium.

In some embodiments, the rare-earth doped crystal comprises at least one Nd-doped host material such as Nd:YAG, Nd:YAP, and/or Nd:GdVO4. In alternative embodiments, the gain medium comprises other rare-earth or transition metal dopants, such as Er, Cr, Ho, Yb, Tm. Other suitable host materials may be glass or crystal materials such as KGW, YVO4, YLF, Forsterite, LiCAF, ZBLAN, or other fluoride or silica glasses. Other choices of gain media and host materials are known to the skilled person, e.g. for use at other wavelengths. See for example: W. Koechner, "Solid-State Laser Engineering", Sixth Edition, Springer Inc., 2006.

The laser module may comprise further optical components, such as a lens, a mirror, or other passive or active optical components, e.g. for shaping the optical field. In particular, the laser resonator may comprise a reflective element and a partly reflective output coupler. In some embodiments, the reflective element comprises a mirror. The mirror may be flat, convex, or concave, depending on e.g. the requirement for beam focusing, to overcome high peak powers, etc. In some embodiments, the reflective element is a grating, such as a fiber Bragg grating or a bulk grating.

In some embodiments, the laser module comprises at least one optical pump source for optically pumping the gain medium, e.g. for end-pumping the gain medium. In some embodiments, the pump source comprises one or more laser diodes. Such diodes are inexpensive, mechanically robust, and practically maintenance-free.

As mentioned above, in some embodiments, the dermatological laser apparatus may be configured to selectively deliver laser radiation at different wavelengths. To this end, in some embodiments, the laser module comprises at least two laser sources configured to create laser radiation at respective wavelengths. Alternatively or additionally, the laser module may comprise a nonlinear medium, in particular a nonlinear crystal, for generating laser radiation by a nonlinear interaction between one or more incoming optical fields, e.g. by frequency doubling or by sum or difference frequency generation, e.g. as described in WO 2019/242919, the entire contents of which are hereby included by reference.

In some embodiments, the set of wavelengths, which can selectively be emitted by the laser module, includes a first wavelength and further a second and/or a third wavelength. In some embodiments, the first wavelength is chosen from the range from about 510 nm to about 620 nm, such as from about 510 nm to about 600 nm, e.g. at about 589 nm. Alternatively or additionally, the set of wavelengths includes a second wavelength chosen from the range from about 1020 nm to about 1080 nm and/or a third wavelength chosen from the range from about 1300 nm to about 1350 nm, e.g. at about 1319 nm. In other embodiments, additional or alternative wavelengths may be used. In one embodiment, the set of wavelengths includes the first and third wavelengths; in one embodiment, the set of wavelengths includes the first, second and third wavelengths.

As mentioned above, in some embodiments, the laser module is configured to create laser radiation that includes more than one wavelength of the set of wavelengths.

In some embodiments, the first wavelength is chosen from the range from about 900 nm to about 980 nm, e.g. the first wavelength may be chosen to be 946 nm. The second wavelength may be chosen from the range from about 1350 nm to about 1650 nm or from the range from about 1500 nm to about 1600 nm. For example, the second wavelength may be chosen to be 1550 nm. For example, the first wavelength may be chosen to be 1550 nm, e.g. using an Er:glass gain medium, and the second wavelength may be chosen to be 946 nm, e.g. using an Nd:YAG gain medium, thus resulting in a third wavelength of about 587 nm. Alternatively, an Er:fibre or Cr+4 such as Cr:YAG gain medium or other suitable gain medium may be used to provide a first wavelength in the range 1350 nm - 1650 nm, and an Nd:xxx gain medium or other suitable gain medium may be used to provide a second wavelength in the range 900 nm - 950 nm (e.g. 914 nm or 946 nm). It will be appreciated that, in some embodiments only the first and third, or only the second and third, or only the first and second wavelengths may be output as a treatment beam, while other embodiments are configured to selectively emit a treatment beam at any one of the first, second and third wavelengths.

In yet other embodiments, the first wavelength is chosen from the range from about 1100 nm to about 1370 nm, such as between 1285 nm and 1370 nm. The second wavelength may be chosen from the range from about 1030 nm to about 1080 nm, e.g. 1064 nm or 1030 nm or even 1080 nm. For example, a Cr+4 such as a Cr:Forsterite (tunable) medium or another suitable gain medium may be used to provide a wavelength in the range 1100 nm - 1370 nm and an Nd-doped gain medium or other suitable medium may be used to provide a wavelength in the range 1030 nm-1080 nm.

The different wavelengths may be used for the treatment of respective skin conditions as they may have different ways of interacting with the skin layers due to different absorption/penetration of the wavelengths into the layers. For instance, light with wavelengths in the range from about 570 nm to about 600 nm may be used for treatment of minor vessels, red discoloration of skin, hyperpigmentation, and to stimulate collagen growth. Light with wavelengths of about 1064 nm or about 1079 nm has a very good penetration due to the low absorption in melanin, hemoglobin and water and may be used to stimulate collagen growth and treat deeper-lying vessels. Light with wavelengths of about 1319 nm or 1350 nm has a good penetration, but higher absorption in water and fatty tissue than, e.g. 1064 nm or 1079 nm light, and may be used to improve skin elasticity and to stimulate collagen growth.

A dermatological laser apparatus capable of including multiple wavelengths at respective relative amounts allows a reduction of the treatment time and provides a flexible, cost-efficient apparatus suitable for multiple types of treatment.

Generally, in the context of a dermatological laser apparatus for skin treatment, there often is a desire or need for providing the treatment beam as short bursts. To this end, the dermatological laser apparatus may include a mechanical shutter, an electro-optical and/or acousto-optical device, or circuitry for adjusting the relative timing of pulsed optical fields fed into a nonlinear medium, e.g. as described in WO 2019/242919.

In some embodiments, the dermatological laser apparatus is configured to irradiate the target skin area in bursts of pulsed light, e.g. bursts of pulsed laser light, each burst having a duration in a range from about 0.5 ms to about 1000 ms, such as from about 0.5 ms to about 900 ms, such as from about 0.5 ms to about 800 ms, such as from about 0.5 ms to about 600 ms, such as from about 0.5 ms to about 400 ms, such as from about 0.5 ms to about 300 ms, such as from about 10 ms to about 200 ms, or even from about 20 ms to about 100 ms, or from about 10 ms to about 40 ms, or from about 0.5 ms to about 40 ms, such as from about 1 ms to about 20 ms, such as from about 0.5 ms to about 4 ms. The burst repetition rate may vary considerably and may, in some embodiments, be selected in the range from 0.1 Hz to 20 Hz, such as from 0.1 Hz to 10 Hz, such as from 0.1 Hz to 5 Hz. In some embodiments, even lower or higher repetition rates may be desirable.

In some embodiments, each burst includes a pulse train of individual laser pulses emitted at a pulse repetition rate. The pulse repetition rate between the individual pulses of a burst may be selected in the range from 0.1 kHz to 500 kHz, such from 1 kHz to 100 kHz, such as from 5 kHz to 50 kHz, such as from 10 kHz to 20 kHz. It will be appreciated that the choice of pulse repetition rate may depend on the components of the dermatological laser apparatus. Each pulse may have a pulse duration of between 10 ns and 100 ns, such as between 20 ns and 80 ns, such as between 50 ns and 70 ns, or another suitable pulse duration, smaller than the burst duration.

In some embodiments, the dermatological laser apparatus is configured to deliver a total radiant exposure of the target area in a range of about 5 J/cm2 to about 150 J/cm2 for each distinct wavelength. In some embodiments, the dermatological laser apparatus is configured to deliver a total radiant exposure of the target area in a range of about 5 J/cm2 to about 100 J/cm2 per distinct wavelength, for example in the range from about 5 J/cm2 to about 60 J/cm2 for each distinct wavelength.

In some embodiments, the radiation-delivery device is configured to scan the treatment beam in a scanning pattern of individual target skin areas covering a treatment area. To this end, the dermatological laser apparatus comprises one or more scanning mirrors, which may e.g. be arranged in the radiation-delivery device or otherwise. The scanning pattern may be pre-set or selectable among a number of pre-programmed patterns, or even be directly programmable, e.g. by a user of the dermatological laser apparatus. Accordingly, different modes of operation may employ different scan patterns. In some embodiments, the scanning pattern is a pattern of individual treatment spots. Each treatment spot may be defined by a beam diameter of the treatment beam, e.g. by a beam waist of the treatment beam. The treatment area may be square or otherwise. For example, the scan pattern may include between 3 and 15 treatment spots in each direction, such as for example a 5×5 pattern, or for example a 6×6 pattern, or for example a 7×7 pattern, or for example a 10x10 pattern or otherwise. Other examples include a pattern with variable spot density in the range of 3-to-15 by 3-to-15 spots. However, other scan patterns, spot densities and/or number of treatment spots in a scan patterns may be used.

FIG. 1 schematically illustrates a block diagram of an embodiment of a dermatological laser apparatus.

The apparatus, generally designated by reference numeral 1, comprises a main unit 11 and a hand-held radiation-delivery device 12. The hand-held radiation-delivery device 12 is connected to the main unit 11 via a cable 13 that may accommodate one or more optical fibers and/or one or more electric connections.

The main unit 11 and the hand-held radiation-delivery device 12 may each have a separate housing and may be implemented as separate units that can be manipulated and moved separately from each other, at least within the restrictions imposed by the cable 13.

The main unit 11 comprises a laser module 111, a control unit 112 and a user interface 113. The user interface 113 and the laser module 111 are operationally coupled to the control unit 112. The control unit 112 may further be operationally coupled to the hand-held radiation-delivery device 12, e.g. via the cable 13, via a wireless connection, and/or otherwise.

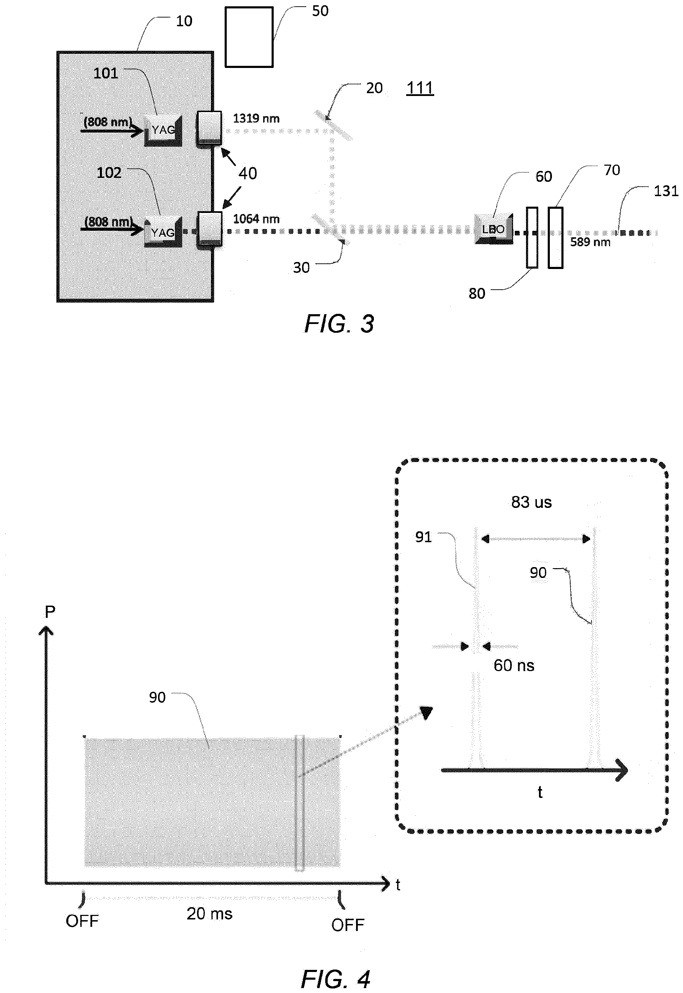

The laser module 111 is configured to create treatment laser radiation at one or more suitable wavelengths as described herein. To this end, the laser module may include one or more suitable laser sources and, optionally other optical components such as a non-linear crystal, one or more lenses, one or more beam splitters, one or more filters, and/or the like. An example of a laser module will be described below with reference to FIG. 3. Further examples of suitable laser sources have been described above and/or are disclosed in WO 2019/242919.

The control unit 112 may comprise a suitably programmed processor, e.g. a microprocessor, an ASIC, or other suitably configured circuitry for controlling operation of the laser module 111, the hand-held radiation-delivery device 12 and the user interface 113. To this end, the control unit 112 may further receive input from the laser module 111, the hand-held radiation-delivery device 12 and/or the user interface 113. The control unit 112 may be implemented as single circuit or as multiple separate components that may be communicatively coupled with each other.

The user interface 113 may comprise any suitable circuitry or device for receiving user inputs and/or for providing user-perceptible outputs. For example, the user interface may include a touch screen and/or a display and/or one or more user-input elements, such as one or more buttons, dials, knobs, joysticks, foot switch, voice input devices, etc. The user interface may comprise one or multiple devices. The user interface 113 may further display or otherwise output information pertinent to the ongoing treatment, e.g. including a measured skin temperature as described herein.

A user of the dermatological laser apparatus may use the user interface 113 to input the patient's skin type and, optionally, other treatment parameters, such as selecting a type of treatment, beam parameters of the treatment laser beam, e.g. wavelength, intensity, pulse duration, scan patterns, etc. The selected treatment parameters define the mode of operation of the dermatological laser apparatus. The control unit 113 is configured to control operation of the laser module and of the hand-held radiation-delivery device 12 based on the user-selected treatment parameters. The user interface 113 may further include a beam activation interface, e.g. a button foot pedal, etc. allowing the user to activate emission of the treatment laser beam.

The treatment laser radiation created by the laser module 111 is fed into a radiation-receiving end of an optical fiber accommodated within the cable 13. The optical fiber has a radiation-deliver end that is coupled to the hand-held radiation-delivery device 12. The hand-held radiation-delivery device 12 thus receives laser radiation from the laser module 111 via the optical fiber of cable 13 and is configured to irradiate a user-selectable target area of the surface 2 of the skin to be treated. The hand-held radiation-delivery device 12 may further receive control signals from the control unit 112.

The hand-held radiation-delivery device 12 may comprise a scanning mirror. The scanning mirror receives the treatment laser radiation via the optical fiber from the laser module 111 and directs the treatment laser radiation via an output port of the hand-held radiation-delivery device as a treatment laser beam 1211 towards the skin surface 2. When the dermatological laser apparatus 1 is operated in a scanning mode of operation, the scanning mirror causes the treatment laser beam 1211 to scan across a target area according to a desired scan pattern. It will be appreciated that the hand-held radiation-delivery device 12 may include one or more additional optical elements, such as one or more lenses, one or more filters, one or more shutters and/or the like.

While FIG. 1 shows the laser module 111, the control unit 112 and the user interface 113 as included in the main unit 11, it will be appreciated that one or more of these components may at least in part be accommodated in the hand-held radiation-delivery device 12. For example, the hand-held radiation-delivery device 12 may comprise a part of the user interface, e.g. in the form of an activation button, one or more LED outputs, and/or the like. Similarly, the hand-held radiation-delivery device 12 may include control circuitry for providing local control of components of the hand-held radiation-delivery device. Yet further, the hand-held radiation-delivery device 12 may include one or more optical components, e.g. for shaping, filtering or otherwise affecting the treatment laser radiation. It will generally be appreciated that the distribution of the various components between the main unit and the hand-held radiation-delivery device may, in some embodiments, be different from what is illustrated in FIG. 1.

FIG. 2 schematically illustrates a flow diagram of an embodiment of a process for controlling a dermatological laser apparatus, e.g. the dermatological laser apparatus of FIG. 1.

In initial step S1, the process obtains, in particular receives, information about the patient's skin type. In embodiments, where the apparatus is operable to treat different skin conditions, the process obtains, in particular receives, information of the skin conditions to be treated. The information of the skin condition to be treated may be or include information about the type of treatment to be performed. In subsequent step S2, the process selects, in dependence of the patient's skin type and, optionally, in dependence on the type of skin condition to be treated, relative amounts of laser energy to be delivered at respective ones of a set of laser wavelengths. To this end the process may retrieve the relative amounts from a data storage 1121, where they may be stored in association with respective skin types and, optionally, skin conditions, e.g. in the form of a look-up table as illustrated in table 1 above, or otherwise. In step S3, the process controls the dermatological laser apparatus to create bursts of laser radiation such that each burst comprises the selected relative amounts of laser energy at the respective ones of the set of laser wavelengths. The process may further select a current mode of operation from a plurality of modes of operation of the dermatological laser apparatus. The plurality of modes of operation may be associated with respective types of dermatological treatments. Each mode of operation may be associated with one or more treatment parameters, e.g. wavelength of the treatment laser beam, a delivery pattern of the treatment laser beam (e.g. a single-spot delivery or a particular scan pattern of the beam delivery), and/or the like.

Embodiments of the laser control described herein can be implemented by means of hardware comprising several distinct elements, and/or at least in part by means of a suitably programmed microprocessor. In the apparatus claims enumerating several means, several of these means can be embodied by one and the same element, component or item of hardware. The mere fact that certain measures are recited in mutually different dependent claims or described in different embodiments does not indicate that a combination of these measures cannot be used to advantage.

FIG. 3 schematically illustrates an example of a laser module of an embodiment of a dermatological laser apparatus.

The laser module 111 includes a laser source module 10, two Q-switches 40, a wavelength conversion unit 60 and a shutter 70. The laser module may further include one or more mirrors 20 and a beam combiner 30. The laser module may further comprise an optical filter 80 and a laser control circuit 50. The laser control circuit may be coupled to, or integrated into, the control unit 112 of the embodiment of FIG. 1, or otherwise.

The laser source module comprises two laser sources 101 and 102 operable to emit laser radiation at a first and second wavelength, respectively. In the present example, the laser sources are diode-pumped YAG crystals configured to emit laser radiation at 1319 nm and 1064 nm, respectively. In other embodiments, the laser source module may include alternative or additionally laser sources, e.g. two laser sources that emit pulsed laser light at different wavelengths.

Each of the Q-switches 40 is coupled to a respective one of the diode-pumped YAG crystals and configured to cause the diode-pumped YAG crystals to emit pulsed laser radiation. The Q-switches are controlled by the laser control circuit 50, such that the laser pulses from the respective YAG crystals are synchronized, i.e. aligned in time, or are partly or completely desynchronized, i.e. such that they only partly overlap in time or such that they do not overlap at all.

The pulsed laser light from the laser sources 101 and 102 is combined into a single beam by beam combiner 30, such as a dichroic mirror, which is essentially transparent for light at the wavelength of one of the laser sources while essentially reflecting at the wavelength of the laser light emitted by the other laser source. In this fashion the two beams may be overlaid. Another example of a beam combiner is a dispersing prism. Other examples of beam combiners are reflection gratings and transmission gratings.

The wavelength conversion unit 60 comprises a nonlinear medium for sum frequency generation conversion of the laser radiation from the two laser sources, thereby generating converted laser radiation at a third wavelength. The nonlinear medium receives the combined laser radiation from the two laser sources via the beam combiner 30. The nonlinear medium of wavelength conversion unit 60 may be implemented by a nonlinear lithium triborate (LBO) crystal. In other embodiments other types of nonlinear media may be utilized and/or other nonlinear processes may be employed. In the present example, the conversion unit creates converted laser radiation at a third wavelength of 589 nm, as 589 nm = ( 1/1064nm + 1/1319nm)⁻¹.

When the laser pulses from the two laser sources arrive at the wavelength conversion unit 60 at the same time, i.e. such that the laser pulses from both laser sources coincide (or at least overlap temporally) with each other in the nonlinear medium, the laser pulses from both laser sources can interact with each other in the non-linear medium so as to generate the converted laser radiation. Accordingly, the laser control circuit 50 can control a relative delay between the laser pulses entering the nonlinear medium such that the laser radiation output by the non-linear medium either includes the converted laser radiation resulting from the nonlinear process or not. Moreover, the amount of converted laser radiation depends on the operation conditions of the nonlinear medium and on the amount of temporal overlap of the incoming pulses from the two laser sources 101 and 102. Accordingly, by controlling the relative overlap of the incoming pulses inside the nonlinear medium, the laser control circuit 50 can control the energy of the generated converted laser radiation. The portion of the incoming laser radiation that does not interact in the nonlinear medium passes through the nonlinear medium. Accordingly, the laser control circuit 50 can control the relative amount of laser energy of the converted laser radiation, relative to the amount of laser energy of the laser radiation from the laser sources 101 and 102 that passes through the conversion unit 60 without being converted. In some embodiments, the laser module 111 comprises one or more optical filters 80 that selectively attenuate or even block light at the wavelength of one or both of the laser sources 101 and 102. The one or more optical filters 80 may be selectively movable into and out of the beam path, or otherwise be selectively activatable, thereby adjusting the relative amount of laser energy emitted at the wavelengths of the laser sources 101 and 102 (e.g. 1319 nm and 1064 nm, respectively). Activation of the one or more optical filters 80 may be controlled by the laser control circuit 50 or otherwise.

For example, when the one or more optical filters are operable to block light from one of the laser sources (e.g. light at 1064 nm), the output beam of 13 the laser module 111 may include radiation at the wavelength of the other laser source 102 (e.g. at 1319 nm) and/or light at the wavelength of the converted laser radiation (e.g. at 589 nm). The relative amount of laser energy at these wavelengths can be controlled by the laser control circuit 50 by adjusting the delay between the Q-switches 40. Alternatively, by suitable controlling the one or more optical filters 80 and the Q-switches 40, the laser module 111 may be controlled to emit an output beam that substantially only includes radiation from the laser source 102 (e.g. at 1064 nm) and the converted laser radiation (e.g. at 589 nm). Yet alternatively, by suitable controlling the one or more optical filters 80 and the Q-switches 40, the laser module 111 may be controlled to emit an output beam that substantially only includes radiation from the laser source 102 (e.g. at 1064 nm) and from the laser source 101 (e.g. at 1319 nm) at relative amounts determined by the adjustable one or more optical filters 80. Yet alternatively, by suitable controlling the one or more optical filters 80 and the Q-switches 40, the laser module 111 may be controlled to emit an output beam that includes radiation from both laser sources 101 and 102 (e.g. at 1319 nm and 1064 nm) as well as converted laser radiation (e.g. at 589 nm), at relative amounts determined by the adjustable one or more optical filters 80 and by the relative delay of the Q-switches 40.

FIG. 4 illustrates the emission of bursts of pulsed laser radiation. Referring to FIG. 4 with continued reference to FIGs. 1 and 3, the output beam 131 from the laser module 111 is emitted as short bursts 90 which may be created by operation of the shutter 70. Each burst 90 comprises a sequence of short laser pulses 91.

In some embodiments, each laser pulse 91 may include laser radiation at one, two or three wavelengths, and the relative amount of the laser energy at the respective wavelength may be controlled by the control unit of the laser apparatus, e.g. by controlling the Q-switches 40 of the laser module 111 so as to control the amount of overlap in time of the pulses at the first and the second wavelengths, and/or by controlling the adjustable one or more optical filters 80 so as to selectively attenuate or even block radiation at one or two of the wavelengths. The control unit 112 may control the Q-switches 40 and/or the optical filter(s) 80 directly or the control unit may control the laser control circuit 50 of the laser module 111 to control the Q-switches 40 and/or the optical filter(s) 80. In any event, the control unit 112 may control the relative amounts of laser energy at the various wavelengths responsive to the skin type and, optionally, responsive to the skin condition to be treated as described herein.

In other embodiments, the control unit 112 controls the laser module 111 such that each burst of the output beam 131 includes laser pulses at respective wavelength, e.g. a first number of pulses at the first wavelength, a second number of pulses at the second wavelength and a third number of pulses at the third wavelength. By selecting the first, second and third numbers, and the energy content of the respective pulses, the control unit can control the relative amounts of laser energy at the respective wavelengths within each burst 70.

It should be emphasized that the term "comprises/comprising" when used in this specification is taken to specify the presence of stated features, elements, steps or components but does not preclude the presence or addition of one or more other features, elements, steps, components or groups thereof.

## Claims

1. A dermatological laser apparatus for dermatological treatment of a patient's skin using a treatment laser beam, the dermatological laser apparatus comprising a laser module, a beam delivery device, and a control circuit, wherein the laser module is configured to create bursts of laser radiation, each burst comprising laser radiation at at least one of a set of laser wavelengths, the set comprising two or more laser wavelengths, wherein the beam delivery device is configured to direct the created laser radiation as a treatment laser beam towards a target area of the patient's skin; and wherein the control circuit is configured to:
- obtain information about the patient's skin type,
- select, in dependence of at least the patient's skin type, relative amounts of laser energy to be delivered at respective ones of the set of laser wavelengths, and
- control the laser module to create the bursts of laser radiation such that each burst comprises the selected relative amounts of laser energy at the respective ones of the set of laser wavelengths.

2. The dermatological laser apparatus according to claim 1, wherein the laser module is configured to create each pulsed laser radiation, wherein each burst includes a sequence of laser pulses.

3. The dermatological laser apparatus according to claim 2, wherein the control unit is configured to control the laser module to create the sequence of laser pulses such that each laser pulse comprises the selected respective amounts of laser energy.

4. The dermatological laser apparatus according to claim 2, wherein the control unit is configured to control the laser module to create the bursts of laser radiation such that each burst comprises laser pulses at respective ones of the set of wavelengths, where each burst comprises relative numbers of laser pulses at the respective wavelengths corresponding to the selected relative amounts of laser energy.

5. The dermatological laser apparatus according to claim 1, wherein the control circuit is configured to obtain information of a type of treatment to be performed and to select the relative amount of laser energy in dependence of the patient's skin type and in dependence of the type of treatment to be performed.

6. A method for controlling a dermatological laser apparatus for dermatological treatment of a patient's skin using a treatment laser beam, the method comprising:
- obtaining information about the patient's skin type,
- selecting, in dependence of at least the patient's skin type, relative amounts of laser energy to be delivered at respective ones of a set of laser wavelengths, and
- controlling the dermatological laser apparatus to create bursts of laser radiation such that each burst comprises the selected relative amounts of laser energy at the respective ones of the set of laser wavelengths.
